# EUROPÄISCHE PATENTANMELDUNG

(11) **EP 2 463 652 A2**
(43) Veröffentlichungstag der Anmeldung: **13.06.2012**
(21) Anmeldenummer: 11008752.5
(22) Anmeldetag: 03.11.2011
(51) Int. Cl.: G01N 33/20

(54) **Verfahren und Vorrichtung zur Analyse von Proben von Metallschmelzen**

(30) Priorität: 07.12.2010 DE 102010053710
(71) Anmelder: Heraeus Electro-Nite International N.V., 3530 Houthalen (BE)
(72) Erfinder: Dams, Francis, 3010 Kessel-Lo (BE); Song, Lihuan, 3920 Lommel (BE); Knevels, Johan, 3960 Bree (BE); Broekmans, Gerrit, 3583 Paal (BE)
(74) Vertreter: Kühn, Hans-Christian

(57) **Zusammenfassung**

Die Erfindung betrifft ein Verfahren zur Analyse von Proben von Metallschmelzen, wobei mittels eines eine Probenkammer aufweisenden, als Eintauchlanze ausgebildeten Probennehmers eine Probe aus einer Metallschmelze genommen wird, und besteht darin, dass die Probe aus dem Probennehmer durch eine Transportleitung hindurch in den Wirkungsbereich einer Analyseneinrichtung transportiert wird und dass dort die Probe mittels der Analyseneinrichtung analysiert wird.

## Beschreibung

Die Erfindung betrifft ein Verfahren zur Analyse von Proben von Metallschmelzen, wobei mittels eines eine Probenkammer aufweisenden, als Eintauchlanze ausgebildeten Probennehmers eine Probe aus einer Metallschmelze entnommen wird. Die Erfindung betrifft weiterhin eine Vorrichtung zur Probennahme in Metallschmelzen, mit einem eine Probenkammer aufweisenden, als Eintauchlanze ausgebildeten Probennehmer, wobei die Vorrichtung insbesondere zur Durchführung des erfindungsgemäßen Verfahrens geeignet sein kann.

In Metallschmelzprozessen, insbesondere bei der Herstellung von Gusseisen oder Stahl, sind regelmäßige Analysen der Schmelze notwendig. Dabei ist es für die Wirtschaftlichkeit des Verfahrens notwendig, dass die Analysen in möglichst kurzer Zeit erfolgen können, um ggf. rechtzeitig die Verfahrensführung entsprechend regeln zu können.

Probenanalyseverfahren sind beispielsweise aus EP 563 447 B1 bekannt. Mit der hier beschriebenen Technik kann der Stickstoffgehalt in Metallschmelzen bestimmt werden. Ähnliche Vorrichtungen und Verfahren sind aus EP 307 430 B1 bekannt. Mit der hier beschriebenen Vorrichtung kann insbesondere der Wasserstoffgehalt in Metallschmelzen analysiert werden. WO 2005/059527 A1 offenbart Analyseverfahren und -vorrichtungen für die Metallschmelze, die mit Einmal-Spektrometern arbeiten. Aus US 4,342,633 sind Eintauchsonden zum Einmalgebrauch bekannt, mit denen Temperatur und Sauerstoffgehalt von Metallschmelzen bestimmt werden können. In JP 3071057 A wird eine Probennahmeeinrichtung für Stahlschmelzen beschrieben, bei der die Probe mit Hilfe einer Eintauchlanze genommen wird, wobei die Probe beim Zurückziehen der Lanze aus der Metallschmelze in eine hermetisch abgeriegelte Kammer gebracht wird. Aus DE 33 44 944 A1 ist es bekannt, Proben zu entnehmen, zu Analyselabors zu transportieren und dort die Analyse durchzuführen. Des Weiteren ist eine spektralanalytische Untersuchung von metallurgischen Tauchproben aus DE 32 00 010 A1 bekannt. Dabei wird insbesondere die Probe unter Vakuum bzw. Inertgasatmosphäre gehalten, um eine Oxidation der heißen Probe zu vermeiden.

Aufgabe der vorliegenden Erfindung ist es, die Zeit zwischen Probenentnahme und Analyse weiter zu verkürzen und dabei Verfälschungen der Probe zu vermeiden.

Die Aufgabe wird im Wesentlichen durch die unabhängigen Ansprüche gelöst. Vorteilhafte Ausgestaltungen ergeben sich aus den Merkmalen der abhängigen Ansprüche. Das erfindungsgemäße Verfahren zur Analyse von Proben von Metallschmelzen, insbesondere von Gusseisen-oder Stahlschmelzen, wobei mittels eines eine Probenkammer aufweisenden, als Eintauchlanze ausgebildeten Probennehmers eine Probe aus einer Metallschmelze genommen wird, ist dadurch gekennzeichnet, dass die Probe aus dem Probennehmer durch eine Transportleitung hindurch in den Wirkungsbereich einer Analyseeinrichtung transportiert wird und dass dort die Probe mittels der Analyseeinrichtung analysiert wird. Die Analyseeinrichtung kann insbesondere ein Spektrometer sein. Dabei wird die Probe unmittelbar aus der Eintauchlanze in die (unmittelbar an die Eintauchlanze anschließende) Transportleitung befördert und von dort zur Analyseeinrichtung, so dass erstens ein schneller Transport gewährleistet ist und zweitens äußere Einflüsse auf die Probe weitgehend ausgeschaltet werden. Die Transportleitung kann in der Art einer Rohrpostleitung ausgebildet sein. Vorzugsweise kann die Probe in mehrere (beispielsweise 2 bis 4) massive Teile geteilt werden, wobei die Teilung bereits im Probennehmer erfolgen kann und Teile der Probe in den Wirkungsbereich der Analyseeinrichtung transportiert werden können. Vorzugsweise kann die Probe auch mit einem von der kompletten Probenkammer lösbaren Teil der Probenkammer verbunden werden und mit diesem Teil in den Wirkungsbereich der Analyseneinrichtung transportiert werden. Ebenso kann es vorteilhaft sein, dass die Probe mit der Probenkammer durch die Transportleitung in den Wirkungsbereich der Analyseeinrichtung transportiert wird, dort ein Teil der Probenkammer entfernt und die dadurch frei werdende Oberfläche der Probe analysiert wird. Insbesondere kann es zweckmäßig sein, dass während des Transports der Probe durch die Transportleitung auf die Probe Vakuum oder Inertgas einwirkt. Vorteilhaft kann es auch sein, dass Vakuum oder Inertgas bereits vor der Probennahme zumindest in der Probenkammer erzeugt wird. Ebenso kann es zweckmäßig sein, dass Vakuum oder Inertgas zumindest in der Probenkammer erzeugt und solange aufrechterhalten wird, bis die Probe auf eine Temperatur von ≤ 400 °C abgekühlt ist. Der Transport der Probe kann vorzugsweise mittels Druckgas erfolgen. Ein Transport unter Vakuum (ein Ansaugen der Probe) ist ebenfalls möglich. Als Druckgas kann insbesondere ein Inertgas (z. B. Argon) verwendet werden, das optional durch Druckluft ersetzt werden kann, wenn die Probe auf eine Temperatur von ≤ 400 °C abgekühlt ist.

Die erfindungsgemäße Vorrichtung zur Probennahme in Metallschmelzen, mit einem eine Probenkammer aufweisenden, als Eintauchlanze ausgebildeten Probennehmer, ist dadurch gekennzeichnet, dass die Probenkammer in einer Kartusche angeordnet ist, dass der Probennehmer mit einem ersten Ende einer Transportleitung für die die Probe oder die die Probe beinhaltende Probenkammer aufweisende Kartusche verbunden ist und dass ein zweites Ende der Transportleitung mit einer Analyseeinrichtung verbunden ist. Die Kartusche ist mit ihrer Außenkontur an die Innenkontur der Eintauchlanze und der sich unmittelbar daran anschließenden Transportleitung angepasst und dient dem Transport der Probenkammer. Die Kartusche kann in die Probenkammer integriert sein, zum Beispiel, wenn die Probenkammer mit ihrer Außenkontur an die Innenkontur von Eintauchlanze und Transportleitung angepasst ist. Die Analyseeinrichtung kann insbesondere ein Spektrometer sein. Zweckmäßig ist es, dass die Probenkammer oder ein Teil davon von dem Probennehmer lösbar und durch die Transportleitung transportierbar ist. Des Weiteren ist es vorteilhaft, dass die Transportleitung einen Druckgasanschluss und/oder einen Vakuumanschluss aufweist. Zweckmäßig ist es weiterhin, dass die Probenkammer und/oder die Kartusche einen Vakuumanschluss oder einen Inertgasanschluss aufweist. Mit der erfindungsgemäßen Vorrichtung ist eine schnelle Probennahme und Weiterleitung der Probe zu einer Analyseeinrichtung möglich, ohne dass die Probe dazwischen schädigenden Umwelteinflüssen ausgesetzt wird.

Nachfolgend wird die Erfindung anhand von Zeichnungen beispielhaft erläutert.

In der Zeichnung zeigt
- Figur 1: die schematische Darstellung einer Probennahme- und Messanordnung
- Figur 2: eine ähnliche Darstellung der Probennahme- und Messanordnung
- Figur 3: den Probennehmer und den Transport der Probe und
- Figur 4: einen weiteren Probennehmer.

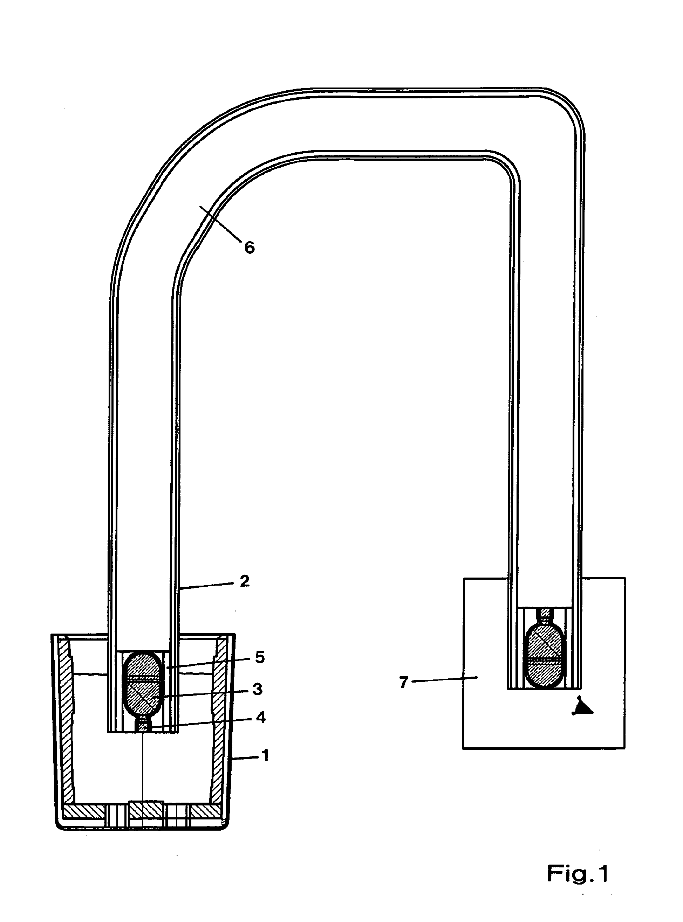

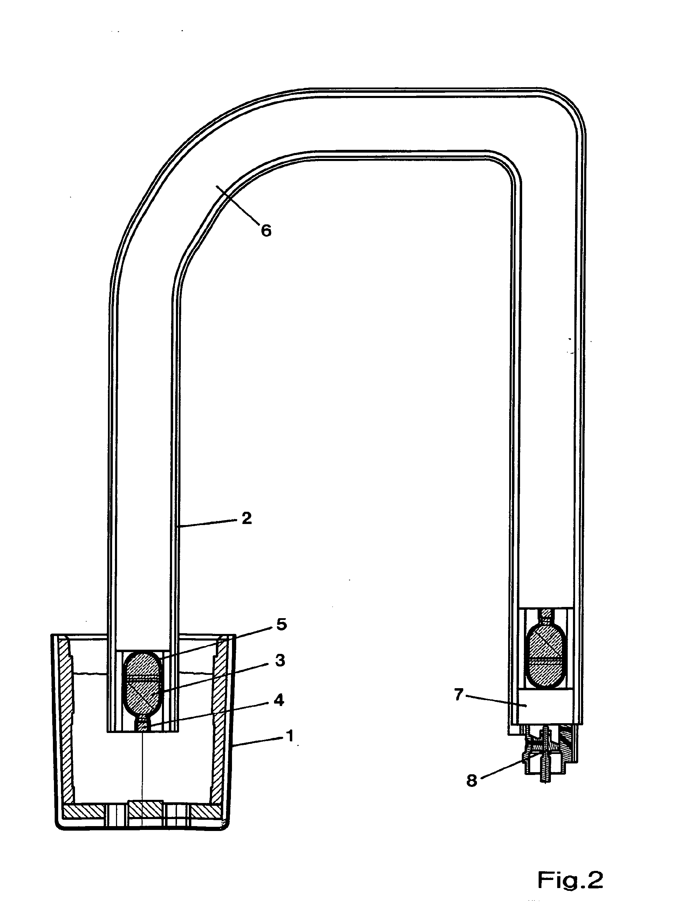

In Figur 1 ist ein Schmelzenbehälter 1 für Stahlschmelze dargestellt, in den eine Eintauchlanze 2, die als Probennehmer dient, eingetaucht wird. Am Eintauchende der Eintauchlanze ist eine Probenkammer 3 angeordnet. Die Probenkammer 3 ist eine sogenannte Lollipop-Probenkammer, also eine Flachprobenkammer mit elliptischem Querschnitt, die an ihrer Eintauchseite einen Einlauf 4 aufweist. Die Probenkammer 3 ist in einer Kartusche 5 fixiert. Mit dieser Kartusche 5 wird die Probenkammer 3 durch die Eintauchlanze 2 und die unmittelbar daran anschließende Transportleitung 6 zu einem Spektrometer 7 transportiert, das als Analyseneinrichtung dient. In Figur 2 ist eine sehr ähnliche Anordnung dargestellt, wobei das Spektrometer 7 mit seinem Funkenstand 8 dargestellt ist. Bei den in den Figuren dargestellten Anordnungen wird die Probenkammer 3 nach ihrem Eintreffen beim Spektrometer 7 geöffnet. Dabei entweicht das vor dem Eintauchen in die Stahlschmelze in die Probenkammer 3 eingeführte Inertgas.

Die Inertgasversorgung der Probenkammer 3 erfolgt in bekannter Weise, wie beispielsweise in DE 32 00 010 A1 dargestellt. Mit dem Inertgas, welches alternativ durch Vakuum ersetzt werden kann, wird verhindert, dass die flüssige Stahlprobe bzw. die erkaltete Stahlprobe bei hohen Temperaturen oxidieren kann.

Beim Erreichen des Spektrometers 7 hat die Probe eine Temperatur von 400 °C weit unterschritten, so dass Inertgas bzw. Vakuum zum Schutz der Probe nicht mehr benötigt werden. Beim Eintreffen der Probenkammer 3 beim Spektrometer 7 wird diese geöffnet. Die Öffnung kann unter anderem auch durch die kinetische Kraft der Probe erfolgen, aber auch mechanisch durch Öffnung der beiden Halbschalen der Probenkammer 3 mittels einer Feder oder durch einen Manipulator, beispielsweise durch eine Trennscheibe oder auch durch Einwirkung von Druckluft. Durch Lösen mindestens einer der beiden Halbschalen der Probenkammer 3 wird eine Oberfläche der Probe der Analyse zugänglich. Da die Probennahme und der Transport der Probe unter Inertgas (beispielsweise Argon) erfolgt ist, wurde eine Oxidation verhindert, so dass die Probe zur Analyse nicht zusätzlich in geeigneter Weise von Oxidation befreit werden muss, eine Probenvorbereitung ist also vor der Analyse nicht mehr notwendig.

Eine derartige Probenzuführung zu einer Analyseeinrichtung erfolgt sehr schnell und direkt, ohne Zwischenstufen, bei denen die Probe auf einem anderen Transportweg transportiert werden muss. Durch die Verbindung der Transportleitung 6 mit der Eintauchlanze 2 und der Analyseneinrichtung ist der Einsatz einer aufwendigen, in unmittelbarer Schmelzennähe zu verwendenden Analyseneinrichtung überflüssig. Die Analyse kann in der Praxis innerhalb von weniger als zwei Minuten erfolgen, da der Transport sehr schnell verläuft und unmittelbar nach der Probennahme beginnt. Darüber hinaus ist eine automatische Probenidentifikation möglich, mit der die Prozessanalyse verbessert werden kann. Die Analyseneinrichtung kann in einem mobilen Labor oder in einem sonstigen festen Labor, beispielsweise in einem Zentrallabor installiert sein. Derartige Labore sind in Stahlwerken ausreichend vorhanden.

In den Figuren ist die Probenkammer 3 als Flachprobenkammer dargestellt, die in einer Kartusche 5 fixiert ist. Die Eintauchlanze 2 hat einen runden Innenquerschnitt, der nahtlos an den ebenfalls runden und gleich großen Innenquerschnitt der Transportleitung 6 anschließt, so dass die Kartusche 5 mit ihrem ebenfalls runden Außenquerschnitt problemlos transportiert werden kann. Statt einer Flachprobenkammer ist es auch denkbar, dass eine Probenkammer mit rundem Querschnitt (senkrecht zur Transportrichtung) verwendet wird. In diesem Fall ist die Kartusche praktisch gleich der äußeren Hülle der Probenkammer 3, so dass die Kartusche in die Probenkammer 3 unmittelbar integriert ist.

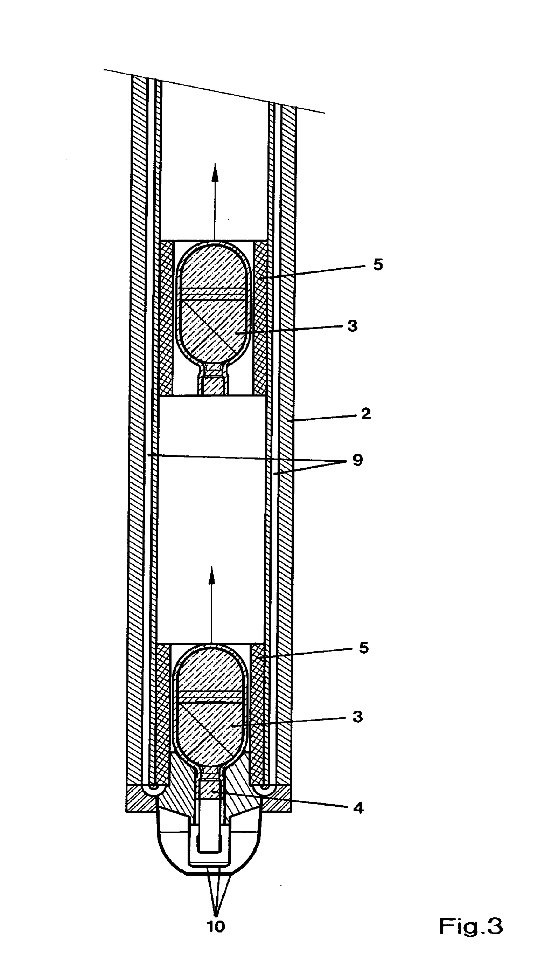

Der Transport der Probenkammer 3 zu der Analyseneinrichtung, die beispielsweise das bereits genannte Spektrometer 7 aufweist, erfolgt mittels Druckluft. Dies ist in Figur 3 in zwei Bewegungsphasen der Probenkammer 3 dargestellt. Die Eintauchlanze 2 geht an ihrem in Figur 3 nicht dargestellten Ende nahtlos in die Transportleitung 6 über. Innerhalb der Wand der Eintauchlanze 2 und gegenbenenfalls der Transportleitung 6 sind Druckgasleitungen angeordnet, mit deren Hilfe ein Gas unter ausreichend hohem Druck gegen die Eintauchseite der Kartusche 5 gedrückt werden kann, so dass diese in Richtung der Analyseneinrichtung transportiert wird. Der freibleibende Querschnitt der Kartusche 5 kann zweckmäßigerweise geschlossen werden, beispielsweise an der dem Eintauchende abgewandten Seite der Kartusche 5 mit einer Scheibe aus einem feuerfesten Material, so dass der Gasdruck effizient den Transport der Probenkammer 3 bewirken kann. In der Eintauchstellung der Probenkammer 3 sind in Figur 3 noch die an der Einlauföffnung 4 der Probenkammer 3 angeordneten Schutzkappen 10 dargestellt, die beim Eintauchen in die Stahlschmelze schmelzen oder aufgelöst werden, so dass die Stahlschmelze in die Probenkammer 3 einfließen kann.

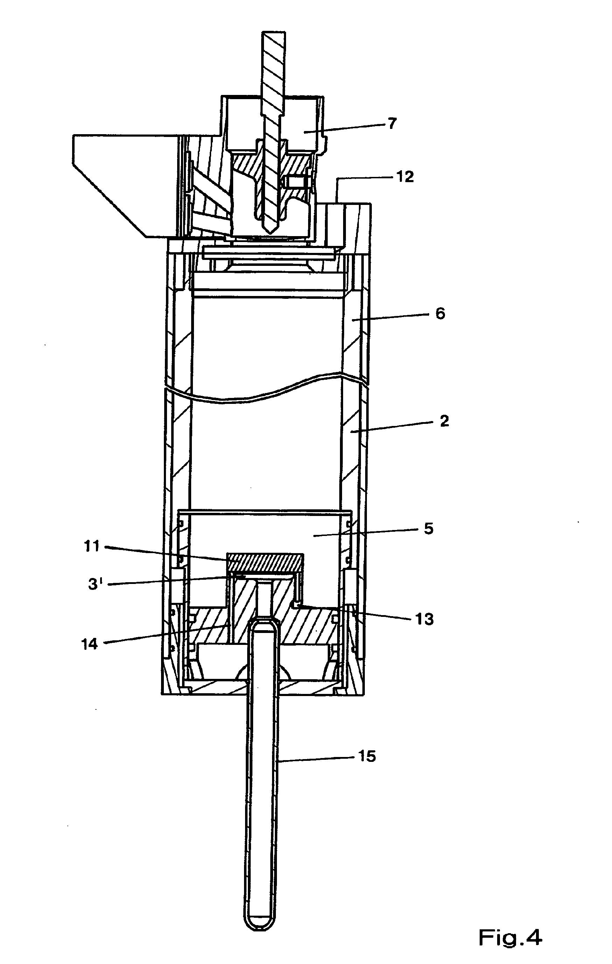

In Figur 4 ist eine weitere mögliche Ausführungsform der Erfindung dargestellt. Im unteren Teil der Figur ist die Eintauchlanze 2 und im oberen Teil der Figur die in die Analyseneinrichtung mündende Transportleitung 6 dargestellt. Die Probenkammer 3' ist als Flachprobenkammer ausgebildet, deren größere Ausdehnung senkrecht zur Eintauchrichtung verläuft. Die Probenkammer 3' ist an ihrem dem Eintauchende abgewandten Ende mit einem entfernbaren Deckel 11 ausgestattet, der nach Eintreffen der Probenkammer 3' am Spektrometer 7 entfernt wird. Das Spektrometer 7 enthält einen Funkenstand, mit dessen Hilfe die Probe an ihrer nach Entfernung des Deckels 11 frei zugänglichen Oberfläche analysiert wird. Die Analyseneinrichtung enthält eine Gaseinlass 12. Eine Gasleitung 13 ist zur Einleitung des Inertgas-Druckgases in der Eintauchlanze 2 an ihrem Eintauchende vorgesehen. Dort ist auch eine Gaszufuhrleitung 14 für die Einleitung von Inertgas in die Probenkammer angeordnet. Die Probenkammer selbst wird durch ein Einlaufrohr 15 aus Quarzglas befüllt.

Die Einzelteile der Vorrichtung sind aus den bei Probennehmern üblichen Materialien ausgebildet.

## Patentansprüche

1. Verfahren zur Analyse von Proben von Metallschmelzen, wobei mittels eines eine Probenkammer aufweisenden, als Eintauchlanze ausgebildeten Probennehmers eine Probe aus einer Metallschmelze genommen wird, **dadurch gekennzeichnet, dass** die Probe aus dem Probennehmer durch eine Transportleitung hindurch in den Wirkungsbereich einer Analyseneinrichtung transportiert wird und dass dort die Probe mittels der Analyseneinrichtung analysiert wird.

2. Verfahren nach Anspruch 1, **dadurch gekennzeichnet, dass** als Analyseneinrichtung ein Spektrometer verwendet wird.

3. Verfahren nach Anspruch 1 oder 2, **dadurch gekennzeichnet, dass** die Probe im Probennehmer geteilt und Teile der Probe in den Wirkungsbereich der Analyseneinrichtung transportiert werden.

4. Verfahren nach Anspruch 1 oder 2, **dadurch gekennzeichnet, dass** die Probe mit einem von der kompletten Probenkammer lösbaren Teil der Probenkammer verbunden wird und mit diesem Teil in den Wirkungsbereich der Analyseneinrichtung transportiert wird.

5. Verfahren nach Anspruch 1 oder 2, **dadurch gekennzeichnet, dass** die Probe mit der Probenkammer durch die Transportleitung in den Wirkungsbereich der Analyseneinrichtung transportiert wird, dort ein Teil der Probenkammer entfernt und die dadurch frei werdende Oberfläche der Probe analysiert wird.

6. Verfahren nach einem der Ansprüche 1 bis 5, **dadurch gekennzeichnet, dass** während des Transports der Probe durch die Transportleitung auf die Probe Vakuum oder Inertgas einwirkt.

7. Verfahren nach einem der Ansprüche 1 bis 6, **dadurch gekennzeichnet, dass** Vakuum oder Inertgas bereits vor der Probennahme zumindest in der Probenkammer erzeugt wird.

8. Verfahren nach einem der Ansprüche 1 bis 7, **dadurch gekennzeichnet, dass** Vakuum oder Inertgas zumindest in der Probenkammer erzeugt und solange aufrechterhalten wird, bis die Probe auf eine Temperatur von kleiner oder gleich 400 °C abgekühlt ist.

9. Verfahren nach einem der Ansprüche 1 bis 8, **dadurch gekennzeichnet, dass** der Transport der Probe mittels Druckgas erfolgt.

10. Verfahren nach Anspruch 9, **dadurch gekennzeichnet, dass** als Druckgas ein Inertgas verwendet wird, das optional durch Druckluft ersetzt werden kann, wenn die Probe auf eine Temperatur von kleiner oder gleich 400 °C abgekühlt ist.

11. Vorrichtung zur Probennahme in Metallschmelzen, mit einem eine Probenkammer aufweisenden, als Eintauchlanze ausgebildeten Probennehmer, **dadurch gekennzeichnet, dass** die Probenkammer in einer Kartusche angeordnet ist, dass der Probennehmer mit einem ersten Ende einer Transportleitung für die die Probe oder die die Probe beinhaltende Probenkammer aufweisende Kartusche verbunden ist und dass ein zweites Ende der Transportleitung mit einer Analyseneinrichtung verbunden ist.

12. Vorrichtung nach Anspruch 11, **dadurch gekennzeichnet, dass** die Analyseneinrichtung ein Spektrometer ist.

13. Vorrichtung nach Anspruch 11 oder 12, **dadurch gekennzeichnet, dass** die Probenkammer oder ein Teil davon von dem Probennehmer lösbar und durch die Transportleitung transportierbar ist.

14. Vorrichtung nach einem der Ansprüche 11 bis 13, **dadurch gekennzeichnet, dass** die Transportleitung einen Druckgasanschluss oder einen Vakuumanschluss aufweist.

15. Vorrichtung nach einem der Ansprüche 11 bis 14, **dadurch gekennzeichnet, dass** die Probenkammer oder die Kartusche einen Vakuumanschluss oder einen Inertgasanschluss aufweist.
